# EUROPEAN PATENT APPLICATION

(11) **EP 3 576 099 A1**
(43) Date of publication of application: **04.12.2019**
(21) Application number: 18174612.4
(22) Date of filing: 28.05.2018
(51) Int. Cl.: G16H 50/30, G16H 20/30, G16H 20/70

(54) **SYSTEM FOR THE PRESENTATION OF ACOUSTIC INFORMATION**

(71) Applicant: Max-Planck-Gesellschaft zur Förderung der Wissenschaften e.V., 80539 München (DE); Universidad Pública de Navarra / Public University of Navarre, 31006 Pamplona-Navarre (ES); Technische Universität Graz, 8010 Graz (AT)
(72) Inventor: Villringer, Prof. Dr., Arno, Berlin 14195 (DE); Meyer. Dr., Lars, Leipzig 04275 (DE); Friederici, Prof. Dr. Dr. h.c, Angela D., Leipzig 04105 (DE); Nikulin, Prof. Dr., Vadim, Berlin 14169 (DE); Gómez, Assoc. Prof., Marisol, 31192 Mutiva, Navarra (ES); Scherer, Assoc. Prof. Dipl. Ing. Dr., Reinhold, 8045 Graz (AT); Vidaurre, Dr., Carmen, 31007 Pamplona, Navarra (ES)
(74) Representative: Hannke, Christian

(57) **Abstract**

System for the presentation of acoustic information comprising an audio unit, which presents acoustic information to a user, at least one detection unit configured to detect at least one biological marker of the user and a processor unit configured to process a signal from the detection unit regarding the detected biological marker, wherein multiple presentation parameter of the acoustic information can continuously be adapted by the processor unit according to at least one continuously detected biological marker corresponding to a cognitive state of the user, wherein the at least one detection unit is a non-invasive neuronal activity detection unit, which continuously detects multiple biological markers in the form of neuronal activity markers.

## Description

The invention relates to a system for the presentation of acoustic information comprising an audio unit, which presents acoustic information to a user, at least one detection unit configured to detect at least one biological marker of the user and a processor unit configured to process a signal from the detection unit regarding the detected biological marker.

Acoustically presented information is at the core of human communication. The human brain has a limited capacity for the processing of auditory information. Further, like all cognitive abilities, the ability of the human brain to process auditory information varies from moment to moment. The attention of a listener to speech may, for instance, be distracted momentarily. Further, the listener may also be too tired to follow very fast or very slow speech.

The capacity for the processing of auditory information is limited even further in the aging population and in listeners suffering from mild cognitive impairment (MCI), decreasing their quality of life and hindering their social inclusion.

MCI mainly affects people over the age of 65 years (65+). It is estimated that 3% to 19% of adults 65+ meet the criteria for MCI. In 2015 the EU-28 population was claimed to be 508,5 million, whereas elderly people with 65+ years of age made up 18,9%, which was 0,4% more than the year before and even 2,3% more than in 2005. The number of people 65+ will rise further in the next years. In absolute numbers this means that currently between 3 and 20 million people (in average about 10 million) are affected with MCI in Europe. Approximately 60-65% of people with MCI will develop clinical dementia during their life. Currently, there is not an accepted treatment to stop or decelerate the progression of MCI to dementia. People affected with MCI have a high demand on support and therefore the health care costs increase dramatically. Direct medical costs for MCI patients are 44% higher than for healthy people.

While human speakers usually adapt naturally to decreases in the listeners' receptive abilities, these decreases are a problem that drastically limit the effectiveness of current digital speech systems. To facilitate the delivery of auditory information, an ideal speaker would need to adapt not only to the presence of patients' communication deficits, but also to their ever-changing brain state, directly relating to the ability to process the presented information. However, the current digital speech systems present speech at a fixed speed, with a fixed loudness and at a fixed information rate. The burden of comprehension is, therefore, put entirely on the side of the listener.

Object of the invention is therefore to provide a system for the presentation of acoustic information that overcomes the above-mentioned disadvantages.

The problem is addressed by a system for the presentation of acoustic information comprising an audio unit, which presents acoustic information to a user, at least one detection unit configured to detect at least one biological marker of the user and a processor unit configured to process a signal from the detection unit regarding the detected biological marker; wherein multiple presentation parameters of the acoustic information can continuously be adapted by the processor unit according to at least one continuously detected biological marker, wherein the at least one detection unit is a non-invasive neuronal activity detection unit, which continuously detects multiple biological markers in the form of neuronal activity markers corresponding to at least one brain process of the user.

Preferably the non-invasive neuronal activity detection unit measures the neural activity in a non-invasive manner.

According to a preferred embodiment at least one presentation parameter of the acoustic information is continuously adapted by the processor unit according to at least one continuously detected biological corresponding to a cognitive state of the user. According to a further preferred embodiment multiple presentation parameters of the acoustic information are continuously adapted by the processor unit according to at least one continuously detected biological corresponding to a cognitive state of the user.

The core idea is therefore to shape the presented acoustical information continuously over time such that it would fit the current cognitive state of the user, with the aim of maximizing the amount of information received without changing the listener's state. This means that the same acoustic information could be presented in many different ways, adjusting to the singular current state of the user. Due to the continuous detection of multiple biological markers in the form of neuronal activity markers corresponding to at least one brain process of the user, the system detects multiple brain processes in parallel. There is an interplay between the listener's cognitive state or brain state and the acoustic speech presentation; thus, brain states affect presentation of acoustic information, and acoustic information will affect the brain state with the intention to increase processing of information. Advantageously the system progressively tracks the brain state and only adapts the presentation parameter of the acoustic information when the state is non-optimal. Further, an adaption of the presentation parameter may be reversed if the brain state returns to the optimal state.

Advantageously the audio unit comprises a speech generator. Further, it may be preferable to implement text to speech technologies in the audio unit. Various formats of text may be presented to the user in an acoustical manner. Alternatively, the audio unit may be configured to replay recorded audio messages. Further, the audio unit may be configured to enable human-to-human communication, for instance by phone or internet services. It is conceivable that beyond the adaptation of the presentation parameter, which is controllable by the processor in such a case, the interlocutor is provided with a signal in case the current cognitive state of the user is not fit to receive the information in an optimal manner. The interlocutor may adapt his speech accordingly. This is particularly of interest for caretakers, doctors dealing with MCI-patients from a distance.

According to a preferred embodiment the non-invasive neuronal activity detection unit is non-invasive brain activity detection unit in the form of an electroencephalography (EEG) unit. EEG is an electrophysiological monitoring method to record electrical activity of the brain. It is the summation of potential shifts that are generated by dendritic ion currents of millions of apical cortical neurons. Numerous short- and long-distance brain networks are active at any given time. EEG patterns result from the superposition of the activity of cortical neurons that are modulated by cognitive activities and involved in short and long-distance network. The non-invasive EEG does not require any invasive procedures.

Advantageously the non-invasive neuronal activity detection unit comprises sensors which may be placed along the scalp. The sensors may be incorporated in a cap the user can wear or in other devices for instance the headphones the user is wearing.

According to a preferred embodiment the presentation parameter is adapted according to detected neuronal activity markers, corresponding to brain processes which relate to language comprehension. Preferably the adaptation of the presentation parameter is based on the detection of neuronal activity markers corresponding to at least three brain processes, namely to a domain-general brain process, to a speech acoustic-specific brain process and to a meaning-dedicated brain process. Advantageously, the domain-general brain process is employed for the monitoring of the user's ability to attend to the acoustic information. Preferably, the speech acoustic-specific brain process is employed for the monitoring of the user's ability to track fine-grained acoustic details. Preferably, the meaning-dedicated brain process is employed for the monitoring of the user's ability to understand the meaning of the acoustic information.

Neurolinguistic models of language dissect the language comprehension process into a broad range of domain-general, low-level sensory, and high-level functions. Each available model focuses only on specific cognitive functions from this range, for instance, acoustic speech perception, cortical networks of production and comprehension systems, differences between languages in electrophysiological language processing, abstract linguistic processing, or the understanding of meaning. In contrast, the advantageous embodiment quantifies the ability of the listener to extract information from the auditory input by considering all three main facets of the language comprehension process, attending the speech, tracking the acoustic details, and understanding the speech, as sub-functions that aim for the goal of language comprehension.

Preferably, both evoked responses and ongoing neuronal oscillations recorded with EEG will be employed for the monitoring of said neuronal activity markers.

According to a further preferred embodiment, the processing unit comprises a comparison unit which compares the signal associated to a detected biological marker with a default state of the biological marker, wherein the presentation parameter of the acoustic information can be continuously adapted by the processor unit due to a deviation of the detected biological marker from a preset default state. Thus, a once changed presentation parameter may be reversed in case the brain state, respectively the corresponding biological marker, returns to the optimal or default state.

Preferably, the parameters of such a default state of the neuronal activity markers of attentiveness, tracking, and comprehension are established at a subject-specific level. Such a subject-specific level may be derived from the user's history and / or from a specific hypothesis. Such a hypothesis could be derived from using a value of a neuronal activity marker from a healthy person and an approximation of this value for the MCI-patient. By deriving from the user's history, the neuronal activity markers in an optimal state, for instance a full attention, are recorded and used as the default value. The neuronal activity markers of attentiveness, tracking, and comprehension are, therefore, continuously tracked and quantified on how much they deviate from the optimal state. Depending on the deviation, appropriate changes in the acoustically presented information are issued.

According to a preferred embodiment the neuronal activity markers corresponding to a domain-general brain process, in particular the attention of the user, comprise the N100 component of the auditory evoked response and power changes in the alpha frequency band of the neural oscillations. According to a further preferred embodiment the neuronal activity markers corresponding to a domain-general brain process, in particular the attention of the user, consist of the N100 component of the auditory evoked response and the power changes in the alpha frequency band of the neural oscillations. The N100 amplitude is considered by a person skilled in the art as a reliable single-trial marker of attentive word processing. In addition to the N100 amplitude, oscillatory power changes in the alpha frequency band are a consistent marker of auditory attention across tasks. The alpha frequency band contains alpha waves which are neural oscillations in the frequency range of 7.5-12.5 Hz. Alpha-band power is enhanced under the suppression of to-be-ignored stimuli and elevated during suboptimal task performance.

According to a preferred embodiment the effect of attention is quantified on the amplitude of the N100, wherein multivariate techniques are preferably used for the extraction of the strongest evoked responses. For this, linear discriminant analysis is preferably used, which is commonly utilized for the extraction of single-trial evoked responses. For the oscillatory power changes in the alpha frequency band evoked by auditory attention, common spatial patterns algorithms are preferably used, which provide the mathematically strongest discrimination in oscillatory power between two conditions.

According to a further preferred embodiment the neuronal activity markers corresponding to speech acoustic-specific brain processes, in particular the track of acoustic details, comprise the phase-locking of the theta-band frequency neural oscillations and the phase-locking of the delta-band frequency neural oscillations. According to a preferred embodiment the neuronal activity markers corresponding to speech acoustic-specific brain processes, in particular the track of acoustic details, consist of the phase-locking of the theta-band frequency neural oscillations and the phase-locking of the delta-band frequency neural oscillations. Thus, the listeners' ability to track fine-grained acoustic details of speech will be monitored via the phase-locking of their theta and delta-band frequency neural oscillations (4-8 Hz and 0.5-4 Hz, respectively) to the acoustic amplitude modulations of the syllable rhythm and pitch contour of speech. Importantly, synchronicity between speech and neural oscillations, as recorded with EEG, has been shown to be a mechanism underlying perceptual intelligibility of speech.

According to a further preferred embodiment the neuronal activity markers corresponding to meaning-dedicated brain processes, in particular information processing, comprise the N400 component of the auditory evoked response and the power changes in the beta frequency band of the neural oscillations. According to a further preferred embodiment the neuronal activity markers corresponding to meaning-dedicated brain processes, in particular information processing, consist of the N400 component of the auditory evoked response and the power changes in the beta frequency band of the neural oscillations. Thus, the listeners' ability to understand word meaning will be monitored via the N400 component of the auditory evoked response, which is associated with the understanding of contextual meaning. Concurrently with the N400, language understanding is associated with oscillatory power changes in the beta frequency band (i.e., 15-30 Hz).

Thus, the advantageously chosen three brain processes attend, follow and understand use the markers, N100 amplitude, oscillatory power changes in the alpha frequency band phase-locking of their theta and delta-band frequency neural oscillations the N400 amplitude and the power changes in the beta frequency. These markers are established across a range of different languages for these chosen three brain processes. Advantageously these markers are detected in parallel. Thus, the corresponding neuronal activity markers for the most essential brain processes to language comprehension are captured as a whole. Further, an optimal joint use of the multiple essential neuronal activity markers is ensured.

According to a further preferred embodiment the presentation parameters of the acoustic information comprise the volume of the presentation of the acoustic information and / or the information rate, respectively the speed of the presentation of the acoustic information and / or the intonation of the presentation of the acoustic information and /or rephrasing of the presentation of the acoustic information and / or repetition of the presentation of the acoustic information.

According to a further preferred embodiment a detected lack of attention, i.e. markers of attention are attenuated compared to periods of known states with full attention, leads to an adaptation of at least one presentation parameter to attract attention; such an adaptation may be increasing the volume. Further, if the user returns to full attention, the volume may be decreased accordingly.

According to a further preferred embodiment a detected lack of the ability to follow the speech respectively a lack of the ability to track fine-grained acoustic details leads to a lag of the neuronal activity markers of speech tracking behind the speech stream. This leads to an adaptation of the information rate respectively the speed of the presentation, meaning the information rate is slowed down. Further, if the user returns to an optimal ability to track fine-grained acoustic details the information rate may be accelerated.

According to a further preferred embodiment a detected lack of the ability to understand, respectively to comprehend the acoustic information leads to an attenuation of the corresponding neuronal activity markers of information processing compared to periods of known states with good information processing. This leads to repetition and /or rephrasing of the presentation of the acoustic information.

It is conceivable that the presentation parameters are arranged in a certain preset order, for instance a detected lack of the ability to understand triggers a repetition acoustic information. In case the repeated acoustic information is still not understood the acoustic information is rephrased.

According to a further preferred embodiment at least one detection unit is a muscle activity detection unit, which continuously detects at least one biological marker in the form of muscle activity of the user.

According to a further preferred embodiment at least one detection unit is a pupil dilation detection unit, which continuously detects at least one biological marker in the form of pupil dilation of the user.

According to a further preferred embodiment at least one detection unit is a facial expression detection unit, which continuously detects at least one biological marker in the form of facial expression of the user.

The detection results from the non-invasive neuronal activity detection unit may preferably be combined with results of further detection units like at least one muscle activity detection unit and / or at least one pupil dilation detection unit and / or at least one facial expression detection unit. A consideration of these detected biological markers in combination with the neuronal activity markers may assist in the evaluation of the cognitive states.

According to a further preferred embodiment the system comprises a feedback sensor to detect a feedback from the user, wherein at least one presentation parameter of the acoustic information can be adapted by the processor unit according to a feedback of the user, wherein the feedback sensor is a visual- and /or an acoustic and / or a haptic feedback sensor. It is therefore possible that the user can request an adaptation of the presentation parameters. This may be triggered by an acoustic signal, a visual signal, for instance handwaving or a haptic signal.

According to a further preferred embodiment the non-invasive neuronal activity monitor unit comprises a brain computer interface, which converts the detected EEG signals into EEG-patterns. Preferably this conversion is done by means of at least one feature extraction technique, preferably at least one multivariate feature extraction technique. Since from oscillatory signals different features are extracted than from evoked potentials feature extraction techniques are used to extract features from both oscillatory and evoked signals. Advantageously the EEG-patterns are converted into machine readable signals by means of machine learning, preferably by means of multivariate machine learning and / or pattern recognition algorithms. By using at least one feature extraction techniques the signal to noise ratio of the brain signals is improved.

According to a further preferred embodiment the system comprises a communication unit, which is configured to establish a connection to at least one digital service. Such digital systems may be social networks, news platforms, text message services. The preferable implemented text to speech technologies may then be used to present the relevant tests into acoustic information.

According to a preferred embodiment the system comprises a recording unit. Such a recording unit may record the data concerning the cognitive states of the users depending certain other parameters for example the time of day or the duration of the presentation of acoustic information. Such data may be evaluated by medical persons in combination with diagnostics of MCI patients.

According to a further preferred embodiment the system, in particular the audio unit, the detection unit and the processor unit are incorporated in one device. Further, the comparison unit and /or the feedback sensor and / or the communication unit and / or the recording unit may be incorporated in such a device.

The object is also addressed by a method for the presentation of acoustic information using a system according to any one of the previous claims comprising the following steps:
- continuously detect multiple biological markers corresponding to a cognitive state of the user by at least one detection unit, wherein the at least one detection unit is a non-invasive neuronal activity detection unit, which continuously detects multiple biological markers in the form of neuronal activity markers;
- comparing the signal associated to a detected biological marker with a preset default state of the biological marker by a comparison unit;
- continuously adapt at least one presentation parameter of the acoustic information according to a deviation of the detected biological marker from a preset default state.

According to a preferred embodiment multiple presentation parameters of the acoustic information are continuously adapted according to a deviation of the detected biological marker from a preset default state.

According to a preferred embodiment of the method the non-invasive neuronal activity detection unit is an electroencephalography (EEG) unit. EEG is an electrophysiological monitoring method to record electrical activity of the brain. It is the summation of potential shifts that are generated by dendritic ion currents of millions of apical cortical neurons. Numerous short and long-distance brain networks are active at any given time. EEG patterns result from the superposition of the activity of cortical neurons that are modulated by cognitive activities and involved in short and long-distance network. The non-invasive EEG does not require any invasive procedures.

According to a preferred embodiment of the method the at least one presentation parameter is adapted according to detected neuronal activity markers, corresponding to brain processes which relate to language comprehension. Preferably, the adaptation of the at least one presentation parameter is based on the detection of neuronal activity markers corresponding to at least three brain processes, namely to a domain-general brain process to a speech acoustic-specific brain process and to a meaning-dedicated brain process. Advantageously the domain-general brain process is employed for the monitoring of the user's ability to attend to the acoustic information. Preferably the speech acoustic-specific brain process is employed for the monitoring of the user's ability to track fine-grained acoustic details. Preferably the meaning-dedicated brain process is employed for the monitoring of the user's ability to understand the meaning of the acoustic information.

Preferably both evoked responses and ongoing neuronal oscillations recorded with EEG will be employed for the monitoring of said neuronal activity markers.

According to a preferred embodiment of the method the neuronal activity markers corresponding to a domain-general brain process, in particular the attention of the user, comprise the N100 component of the auditory evoked response and power changes in the alpha frequency band of the neural oscillations. According to a further preferred embodiment the neuronal activity markers corresponding to a domain-general brain process, in particular the attention of the user, consist of the N100 component of the auditory evoked response and the power changes in the alpha frequency band of the neural oscillations.

According to a further preferred embodiment of the method the neuronal activity markers corresponding to speech acoustic-specific brain processes, in particular the track of acoustic details, comprise the phase-locking of the theta-band frequency neural oscillations and the phase-locking of the delta-band frequency neural oscillations. According to a preferred embodiment the neuronal activity markers corresponding to speech acoustic-specific brain processes, in particular the track of acoustic details, consist of the phase-locking of the theta-band frequency neural oscillations and the phase-locking of the delta-band frequency neural oscillations.

According to a further preferred embodiment of the method the neuronal activity markers corresponding to meaning-dedicated brain processes, in particular information processing, comprise the N400 component of the auditory evoked response and the power changes in the beta frequency band of the neural oscillations. According to a further preferred embodiment the neuronal activity markers corresponding to meaning-dedicated brain processes, in particular information processing, consist of the N400 component of the auditory evoked response and the power changes in the beta frequency band of the neural oscillations

Thus, the advantageously chosen three brain processes attend, follow and understand use the markers, N100 amplitude, oscillatory power changes in the alpha frequency band phase-locking of their theta and delta-band frequency neural oscillations the N400 amplitude and the power changes in the beta frequency. These markers are established across a range of different languages for these chosen three brain processes. Advantageously these markers are detected in parallel. Thus, the corresponding neuronal activity markers for the most essential brain processes to language comprehension are captured as a whole. Further, an optimal joint use of the multiple essential neuronal activity markers is ensured.

According to a further preferred embodiment of the method the presentation parameters of the acoustic information comprise the volume of the presentation of the acoustic information, wherein the volume of the presentation of the acoustic information according to the neuronal activity markers correspond to the domain-general brain processes.Thus, a detected lack of attention, i.e. markers of attention are attenuated compared to periods of known states with full attention, leads to an adaptation of the presentation parameters to attract attention; such an adaptation may be increasing the volume. Further, if the user returns to full attention, the volume may be decreased accordingly.

According to a further preferred embodiment of the method the presentation parameters of the acoustic information comprise the information rate of the presentation of the acoustic information, wherein the information rate of the presentation of the acoustic information according to the neuronal activity markers corresponds to the speech acoustic-specific brain process. Thus, a detected lack of the ability to follow the speech respectively a lack of the ability to track fine-grained acoustic details leads to a lag of the neuronal activity markers of speech tracking behind the speech stream. This leads to an adaptation of the information rate, respectively the speed of the presentation, meaning the information rate is slowed down. Further, if the user returns to an optimal ability to track fine-grained acoustic details, the information rate may be accelerated.

According to a further preferred embodiment of the method the presentation parameters of the acoustic information comprise rephrasing of the presentation of the acoustic information and / or repetition of the presentation of the acoustic information, wherein the information rate of the presentation of the acoustic information according to the neuronal activity markers corresponds to the meaning-dedicated brain process. Thus, a detected lack of the ability to understand, respectively to comprehend the acoustic information leads to an attenuation of the corresponding neuronal activity markers of information processing compared to periods of known states with good information processing. This leads to repetition and /or rephrasing of the presentation of the acoustic information.

It is conceivable that the presentation parameters are arranged in a certain preset order, for instance a detected lack of the ability to understand triggers a repetition acoustic information. In case the repeated acoustic information is still not understood the acoustic information is rephrased.

According to a further preferred embodiment of the method the non-invasive neuronal brain activity monitor unit comprises a brain computer interface, which converts the detected EEG signals into EEG-patterns. Preferably this conversion is done by means of at least one feature extraction technique, preferably at least one multivariate feature extraction technique. Since from oscillatory signals different features are extracted than from evoked potentials feature extraction techniques are used to extract features from both oscillatory and evoked signals. Advantageously, the EEG-patterns are converted into machine readable signals by means of machine learning, preferably by means of multivariate machine learning and / or pattern recognition algorithms. By using at least one feature extraction technique the signal to noise ratio of the brain signals is improved.

Further, the method may comprise the same embodiments and advantages as described in conjunction with the above-described image device.

The system and the method optimize the reception of auditory information presented through digital sources. This principle can be applied in nearly any digital service that is based on auditory information; it thus improves the delivery and facilitates both uptake and acceptance of digital services.

Further advantages, aims and properties of the present invention will be described by way of the appended drawings and the following description.

In the drawings:
- Fig. 1: shows a system for the presentation of acoustic information;
- Fig. 2: shows a flow chart of the brain processes involved in language comprehension.

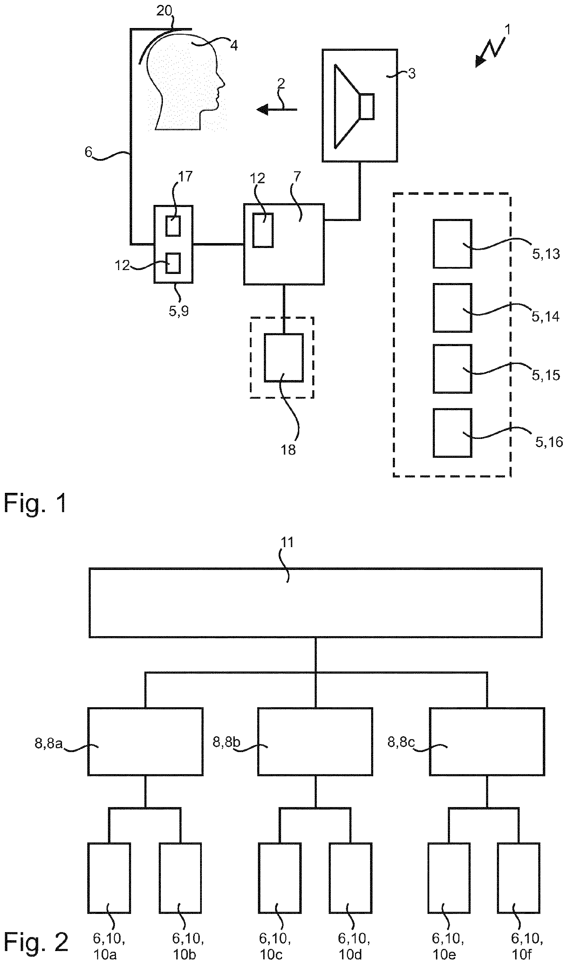

Figure 1 shows a system (1) for the presentation of acoustic information (2) comprising an audio unit (3), which presents acoustic information (2) to a user (4), at least one detection unit (5) configured to detect at least one biological marker (6) of the user (4) and a processor unit (7) configured to process a signal from the detection unit (5) regarding the detected biological marker (6); wherein multiple presentation parameters of the acoustic information (2) can continuously be adapted by the processor unit (7) according to at least one continuously detected biological marker (6) corresponding to a cognitive state (8) of the user (4), wherein the at least one detection unit (5) is a non-invasive neuronal activity detection unit (9), which continuously detects multiple biological markers (6) in the form of neuronal activity markers (10). The system is, therefore, able to monitor multiple brain processes (8) in parallel.

It is conceivable that at least one presentation parameter of the acoustic information (2) is continuously adapted according to a deviation of the detected biological marker (6, 10, 10a-10f) from a preset default state. Optimally multiple presentation parameters of the acoustic information (2) are continuously adapted according to a deviation of the detected biological marker (6, 10, 10a-10f) from a preset default state.

The non-invasive neuronal activity detection unit (9) is an electroencephalography (EEG) unit. To the non-invasive neuronal activity detection unit (9) at least one sensor (20) is connected which may be placed on the scalp of the user (4).

At least one presentation parameter is adapted according to detected neuronal activity markers (6, 10) corresponding to brain processes which relate to language comprehension (11). The adaptation of at least one presentation parameter is based on the detection of neuronal activity markers (6, 10) corresponding to at least three brain processes (8), namely to a domain-general brain process (8a), to a speech acoustic-specific brain process (8b) and to a meaning-dedicated brain process (8c). This is depicted in Figure 2.

The processing unit comprises a comparison unit (12) which compares the signal associated to a detected biological marker (6, 10) with a default state of the biological marker (6, 10), wherein the at least one presentation parameter of the acoustic information (2) is continuously adapted by the processor unit (7) due to a deviation of the detected biological marker (6, 10) from a preset default state. Thus, the system progressively tracks the brain state and only adapts presentation of the acoustic information (2) when the state is non-optimal.

Optionally the system may comprise a communication unit (18), which is configured to establish a connection to at least one digital service. Further optional expansions (marked by the dashed line) are a muscle activity detection unit (13), which continuously detects at least one biological marker (6) in the form of muscle activity of the user (4), a pupil dilation detection unit (14), which continuously detects at least one biological marker (6) in the form of pupil dilation of the user (4) and a facial expression detection unit (15), which continuously detects at least one biological marker (6) in the form of facial expression of the user (4).

Finally, another optional feature is a feedback sensor (16) to detect a feedback from the user (4), wherein at least one presentation parameter of the acoustic information (2) can be adapted by the processor unit (7) according to a feedback of the user (4), wherein the feedback sensor (16) is a visual- and /or an acoustic and / or a haptic feedback sensor.

Further, the non-invasive neuronal activity detection unit (5, 9) comprises a brain computer interface BCI (17), which converts the detected EEG signals into EEG-patterns by means of at least one feature extraction technique, preferably at least one multivariate feature extraction technique. Since from oscillatory signals different features are extracted than from evoked potentials feature extraction techniques are used to extract features from both oscillatory and evoked signals. Preferably, the EEG-patterns are converted into machine readable signals by means of machine learning and / or pattern recognition algorithms.

According to Figure 2 the neuronal activity markers (6,10) corresponding to the domain-general brain process (8a) comprise the N100 component of the auditory evoked response (10a) and the power changes in the alpha frequency band of the neural oscillations (10b). Further, the neuronal activity markers (6, 10) corresponding to speech acoustic-specific brain process (8b) comprise the phase-locking of the theta-band frequency neural oscillations (10c) and the phase-locking of the delta-band frequency neural oscillations (10d). Finally, the neuronal activity markers (8, 10) corresponding to the meaning-dedicated brain process (8c) comprise the N400 component of the auditory evoked response (10e) and the power changes in the beta frequency band of the neural oscillations (10d). Hence, the most crucial EEG markers of language comprehension are used to evaluate the degree of information comprehension. By applying necessary changes to the speech signal these biomarkers are driven into a range associated with optimal language comprehension.

The system (1) monitors multiple brain processes (8) in parallel due to the detection of multiple biological markers (6) in the form of neuronal activity markers (10). The system (1) monitors the attention of the user (4), tracks acoustic details by corresponding brain systems and tracks the listeners' understanding of the meaning of the acoustic stimulus. Thus, the system (1) is a multi-domain parallel tracking system. The list of to-be-tracked markers does not only include neuronal activity markers (10) but is expandable to include additional biomarkers (6) such as muscle activity, pupil dilation, or facial expressions recorded either from sensors on the body directly or from external sensors. The system will not only provide feedback to the user (4), but also to other individuals involved in the communicative setting or discourse.

All the features disclosed in the application documents are claimed as being essential to the invention if, individually or in combination, they are novel over the prior art.

### List of reference numerals

- 1: system
- 2: acoustic information
- 3: audio unit
- 4: user
- 5: detection unit
- 6: biological marker
- 7: processor unit
- 8: cognitive state, brain process
- 8a: domain-general brain process
- 8b: speech acoustic-specific brain process
- 8c: meaning-dedicated brain process
- 9: non-invasive neuronal activity detection unit
- 10: neuronal activity markers
- 10a: N100 component of the auditory evoked response
- 10b: power changes in the alpha frequency band of the neural oscillations
- 10c: phase-locking of the theta-band frequency neural oscillations
- 10d: phase-locking of the delta-band frequency neural oscillations
- 10e: N400 component of the auditory evoked response
- 10d: power changes in the beta frequency band of the neural oscillations
- 11: language comprehension
- 12: comparison unit
- 13: muscle activity detection unit
- 14: pupil dilation detection unit
- 15: facial expression detection unit
- 16: feedback sensor
- 17: brain computer interface
- 19: communication unit
- 20: sensor

## Claims

1. System (1) for the presentation of acoustic information (2) comprising an audio unit (3), which presents acoustic information (2) to a user (4), at least one detection unit (5), configured to detect at least one biological marker (6) of the user (4) and a processor unit (7), configured to process a signal from the detection unit (5) regarding the detected biological marker (6),
**characterized in that**
multiple presentation parameters of the acoustic information (2) can continuously be adapted by the processor unit (7) according to at least one continuously detected biological marker (6) corresponding to a cognitive state (8) of the user (4), wherein the at least one detection unit (5) is a non-invasive neuronal activity detection unit (9), which continuously detects multiple biological markers (6) in the form of neuronal activity markers (10).

2. System according to claim 1,
**characterized in that**
the non-invasive neuronal activity detection unit (9) is an electroencephalography (EEG) unit, wherein at least one presentation parameter is adapted according to detected neuronal activity markers (6, 10), corresponding to brain processes which relate to language comprehension (11), wherein the adaptation of at least one presentation parameter is based on the detection of neuronal activity markers (6, 10) corresponding to at least three brain processes (8), namely to a domain-general brain process (8a), to a speech acoustic-specific brain process (8b) and to a meaning-dedicated brain process.

3. System according to claim 1,
**characterized in that**
the processing unit comprises a comparison unit (12) which compares the signal associated to a detected biological marker (6, 10) with a default state of the biological marker (6, 10), wherein the presentation parameter of the acoustic information (2) is continuously adapted by the processor unit (7) due to a deviation of the detected biological marker (6, 10) from a preset default state.

4. System according to one of the previous claims
**characterized in that**
the neuronal activity markers (6,10) corresponding to the domain-general brain process (8a) comprise the N100 component of the auditory evoked response (10a) and the power changes in the alpha frequency band of the neural oscillations (10b), wherein the neuronal activity markers (6, 10) corresponding to speech acoustic-specific brain process (8b) comprise the phase-locking of the theta-band frequency neural oscillations (10c) and the phase-locking of the delta-band frequency neural oscillations (10d), wherein the neuronal activity markers (8, 10) corresponding to the meaning-dedicated brain process (8c) comprise the N400 component of the auditory evoked response (10e) and the power changes in the beta frequency band of the neural oscillations (10d).

5. System according to one of the previous claims
**characterized in that**
the presentation parameters of the acoustic information (2) comprise the volume of the presentation of the acoustic information (2) and / or the information rate of the presentation of the acoustic information (2) and / or the intonation of the presentation of the acoustic information (2) and /or rephrasing of the presentation of the acoustic information (2) and / or repetition of the presentation of the acoustic information (2).

6. System according to one of the previous claims
**characterized in that**
at least one detection unit (5) is a muscle activity detection unit (13), which continuously detects at least one biological marker (6) in the form of muscle activity of the user (4), wherein at least one detection unit (5) is a pupil dilation detection unit (14), which continuously detects at least one biological marker (6) in the form of pupil dilation of the user (4), wherein at least one detection unit (5) is a facial expression detection unit (15), which continuously detects at least one biological marker (6) in the form of facial expression of the user (4).

7. System according to one of the previous claims
**characterized in that**
the system (1) comprises a feedback sensor (16) to detect a feedback from the user (4), wherein at least one presentation parameter of the acoustic information (2) can be adapted by the processor unit (7) according to a feedback of the user (4), wherein the feedback sensor (16) is a visual- and /or an acoustic and / or a haptic feedback sensor.

8. System according to one of the previous claims 2 to 7
**characterized in that**
the non-invasive neuronal activity detection unit (5, 9) comprises a brain computer interface BCI (17), which converts the detected EEG signals into EEG-patterns by means of at least one feature extraction technique preferably at least one multivariate feature extraction technique, wherein the EEG-patterns are converted into machine readable signals by means of machine learning, preferably by means of multivariate machine learning, and / or pattern recognition algorithms.

9. System according to one of the previous claims
**characterized in that**
the system comprises a communication unit (18), which is configured to establish a connection to at least one digital service.

10. Method for the presentation of acoustic information (2) using a system (1) according to any one of the previous claims comprising the following steps:
- continuously detect multiple biological markers (6) corresponding to a cognitive state (8) of the user (4) by at least one detection unit (5), wherein the at least one detection unit (5) is a non-invasive neuronal activity detection unit (9), which continuously detects multiple biological markers (6) in the form of neuronal activity markers (10, 10a-10f);
- comparing the signal associated to a detected biological marker (6, 10, 10a-10f) with a preset default state of the biological marker (6, 10, 10a-10f) by a comparison unit (12);
- continuously adapt at least one presentation parameter of the acoustic information (2) according to a deviation of the detected biological marker (6, 10, 10a-10f) from a preset default state.

11. Method according to claim 10
**characterized in that**
multiple presentation parameters of the acoustic information (2) are continuously adapted according to a deviation of the detected biological marker (6, 10, 10a-10f) from a preset default state.

12. Method according to one of the claims 10 to 11
**characterized in that**
the non-invasive neuronal activity detection unit (6a) is an electroencephalography (EEG) unit, wherein at least one presentation parameter is adapted according to detected neuronal activity markers (6, 10), corresponding to brain processes which relate to language comprehension (11), wherein the adaptation of at least one presentation parameter is based on the detection of neuronal activity markers (6, 10) corresponding to at least three brain processes (8), namely to a domain-general brain process (8a), to a speech acoustic-specific brain process (8b) and to a meaning-dedicated brain process.

13. Method according to claim 12
**characterized in that**
the presentation parameters of the acoustic information (2) comprise the volume of the presentation of the acoustic information (2), wherein the volume of the presentation of the acoustic information (2) according to the neuronal activity markers (6, 10, 10a, 10b) corresponds to the domain-general brain process (8a).

14. Method according to one of the claims 12 to 13
**characterized in that**
the presentation parameters of the acoustic information (2) comprise the information rate of the presentation of the acoustic information (2), wherein the information rate of the presentation of the acoustic information according to the neuronal activity markers (6, 10, 10c, 10d) corresponds to the speech acoustic-specific brain process (8b).

15. Method according to one of the claims 12 to 14
**characterized in that**
the presentation parameters of the acoustic information (2) comprise rephrasing of the presentation of the acoustic information (2) and / or repetition of the presentation of the acoustic information (2), wherein the information rate of the presentation of the acoustic information (2) according to the neuronal activity markers (6, 10, 10e, 10f) corresponds to the meaning-dedicated brain process (8c).

## Amended claims

### Amended claims in accordance with Rule 137(2) EPC.

1. System (1) for the presentation of acoustic information (2) comprising an audio unit (3), which presents acoustic information (2) to a user (4), at least one detection unit (5), configured to detect at least one biological marker (6) of the user (4) and a processor unit (7), configured to process a signal from the detection unit (5) regarding the detected biological marker (6),
**characterized in that**
multiple presentation parameters of the acoustic information (2) can continuously be adapted by the processor unit (7) according to at least one continuously detected biological marker (6) corresponding to a cognitive state (8) of the user (4), wherein the at least one detection unit (5) is a non-invasive neuronal activity detection unit (9), which continuously detects multiple biological markers (6) in the form of neuronal activity markers (10), wherein at least one presentation parameter is adapted according to detected neuronal activity markers (6, 10), corresponding to brain processes which relate to language comprehension (11), wherein the adaptation of at least one presentation parameter is based on the detection of neuronal activity markers (6, 10) corresponding to at least three brain processes (8), namely to a domain-general brain process (8a) for the monitoring of the user's ability to attend to the acoustic information (2), to a speech acoustic-specific brain process (8b) for the monitoring of the user's ability to track acoustic details of the acoustic information (2), and to a meaning-dedicated brain process for the monitoring of the user's ability to understand the meaning of the acoustic information (2), wherein the processing unit comprises a comparison unit (12), which compares the signal associated to the detected biological markers (6, 10) with a default state of the biological markers (6, 10), wherein said default state refers to a cognitive state (8) of full attention of the user (4), wherein the presentation parameter of the acoustic information (2) is adapted by the processor unit (7) due to a deviation of the detected biological markers (6, 10) from the preset default state.

2. System according to claim 1,
**characterized in that**
the non-invasive neuronal activity detection unit (9) is an electroencephalography (EEG) unit.

3. System according to one of the previous claims
**characterized in that**
the neuronal activity markers (6,10) corresponding to the domain-general brain process (8a) comprise the N100 component of the auditory evoked response (10a) and the power changes in the alpha frequency band of the neural oscillations (10b), wherein the neuronal activity markers (6, 10) corresponding to speech acoustic-specific brain process (8b) comprise the phase-locking of the theta-band frequency neural oscillations (10c) and the phase-locking of the delta-band frequency neural oscillations (10d), wherein the neuronal activity markers (8, 10) corresponding to the meaning-dedicated brain process (8c) comprise the N400 component of the auditory evoked response (10e) and the power changes in the beta frequency band of the neural oscillations (10d).

4. System according to one of the previous claims
**characterized in that**
the presentation parameters of the acoustic information (2) comprise the volume of the presentation of the acoustic information (2) and / or the information rate of the presentation of the acoustic information (2) and / or the intonation of the presentation of the acoustic information (2) and /or rephrasing of the presentation of the acoustic information (2) and / or repetition of the presentation of the acoustic information (2).

5. System according to one of the previous claims
**characterized in that**
at least one detection unit (5) is a muscle activity detection unit (13), which continuously detects at least one biological marker (6) in the form of muscle activity of the user (4), wherein at least one detection unit (5) is a pupil dilation detection unit (14), which continuously detects at least one biological marker (6) in the form of pupil dilation of the user (4), wherein at least one detection unit (5) is a facial expression detection unit (15), which continuously detects at least one biological marker (6) in the form of facial expression of the user (4).

6. System according to one of the previous claims
**characterized in that**
the system (1) comprises a feedback sensor (16) to detect a feedback from the user (4), wherein at least one presentation parameter of the acoustic information (2) can be adapted by the processor unit (7) according to a feedback of the user (4), wherein the feedback sensor (16) is a visual- and /or an acoustic and / or a haptic feedback sensor.

7. System according to one of the previous claims 2 to 6
**characterized in that**
the non-invasive neuronal activity detection unit (5, 9) comprises a brain computer interface BCI (17), which converts the detected EEG signals into EEG-patterns by means of at least one feature extraction technique preferably at least one multivariate feature extraction technique, wherein the EEG-patterns are converted into machine readable signals by means of machine learning, preferably by means of multivariate machine learning, and / or pattern recognition algorithms.

8. System according to one of the previous claims
**characterized in that**
the system comprises a communication unit (18), which is configured to establish a connection to at least one digital service.

9. Method for the presentation of acoustic information (2) using a system (1) according to any one of the previous claims comprising the following steps:
- continuously detect multiple biological markers (6) corresponding to a cognitive state (8) of the user (4) by at least one detection unit (5), wherein the at least one detection unit (5) is a non-invasive neuronal activity detection unit (9), which continuously detects multiple biological markers (6) in the form of neuronal activity markers (10, 10a-10f);
- comparing the signal associated to a detected biological marker (6, 10, 10a-10f) with a preset default state of the biological marker (6, 10, 10a-10f) by a comparison unit (12), wherein said default state refers to a cognitive state (8) of full attention of the user (4);
- continuously adapt at least one presentation parameter of the acoustic information (2) according to a deviation of the detected biological marker (6, 10, 10a-10f) from a preset default state wherein the presentation parameter of the acoustic information (2) is adapted by the processor unit (7) due to a deviation of the detected biological markers (6, 10) from the preset default state;
wherein at least one presentation parameter is adapted according to detected neuronal activity markers (6, 10), corresponding to brain processes which relate to language comprehension (11), wherein the adaptation of at least one presentation parameter is based on the detection of neuronal activity markers (6, 10) corresponding to at least three brain processes (8), namely to a domain-general brain process (8a) for the monitoring of the user's ability to attend to the acoustic information (2), to a speech acoustic-specific brain process (8b) for the monitoring of the user's ability to track acoustic details of the acoustic information (2), and to a meaning-dedicated brain process for the monitoring of the user's ability to understand the meaning of the acoustic information (2).

10. Method according to claim 9
**characterized in that**
the non-invasive neuronal activity detection unit (6a) is an electroencephalography (EEG) unit.

11. Method according to one of the claims 9 to 10
**characterized in that**
the presentation parameters of the acoustic information (2) comprise the volume of the presentation of the acoustic information (2), wherein the volume of the presentation of the acoustic information (2) according to the neuronal activity markers (6, 10, 10a, 10b) corresponds to the domain-general brain process (8a).

12. Method according to one of the claims 9 to 11
**characterized in that**
the presentation parameters of the acoustic information (2) comprise the information rate of the presentation of the acoustic information (2), wherein the information rate of the presentation of the acoustic information according to the neuronal activity markers (6, 10, 10c, 10d) corresponds to the speech acoustic-specific brain process (8b).

13. Method according to one of the claims 9 to 12
**characterized in that**
the presentation parameters of the acoustic information (2) comprise rephrasing of the presentation of the acoustic information (2) and / or repetition of the presentation of the acoustic information (2), wherein the information rate of the presentation of the acoustic information (2) according to the neuronal activity markers (6, 10, 10e, 10f) corresponds to the meaning-dedicated brain process (8c).
